# EUROPEAN PATENT SPECIFICATION

(11) **EP 2 110 430 B1**
(45) Date of publication and mention of the grant of the patent: **09.06.2010**
(21) Application number: 07866380.4
(22) Date of filing: 21.12.2007
(51) Int. Cl.: C12M 1/34, C12N 11/08, G01N 21/77, G01N 33/18

(54) **MEASURING CELL, ANALYSER, PROCESS, AND COMPUTER PROGRAM PRODUCT FOR MEASURING BIOCHEMICAL OXYGEN DEMAND (BOD)**
MESSZELLE, ANALYSEGERÄT, VERFAHREN UND COMPUTERPROGRAMM ZUR MESSUNG EINES BIOCHEMISCHEN SAUERSTOFFBEDARFS
CELLULE DE MESURE, ANALYSEUR, PROCÉDÉ, PROGRAMME D'ORDINATEUR ET SUPPORT DE CE PROGRAMME PERMETTANT DE MESURER DBO

(30) Priority: 28.12.2006 ES 200603300
(43) Date of publication of application: 21.10.2009
(73) Proprietor: Interlab, Ingeniería Electrónica Y De Control, S.A.U., 28050 Madrid (ES)
(72) Inventor: BEDOYA GUTIÉRREZ, Maximino, E-28592 Rivas Vaciamadrid - Madrid (ES); DELGADO ALONSO, Jesús, E-28660 Boadilla Del Monte - Madrid (ES); GARCÍA ARES, Ernesto, E-28018 Madrid (ES); LUIS GARCÍA, José, E-28011 Madrid (ES); ORELLANA MORALEDA, Guillermo, E-28039 Madrid (ES); MORENO BONDI, María Cruz, E-28039 Madrid (ES)
(74) Representative: ABG Patentes, S.L.
(86) International application number: PCT/ES2007/000755
(87) International publication number: WO 2008/081060

(56) References cited:
- DE-A1- 4 301 087
- GB-A- 2 014 979
- KWOK ET AL: "An optical biosensor for multi-sample determination of biochemical oxygen demand (BOD)" SENSORS AND ACTUATORS B, vol. 110, no. 2, 14 October 2005 (2005-10-14), pages 289-298, XP005023955 ISSN: 0925-4005
- LIN ET AL: "Novel BOD optical fiber biosensor based on co-immobilized microorganisms in ormosils matrix" BIOSENSORS & BIOELECTRONICS, vol. 21, no. 9, 15 March 2006 (2006-03-15), pages 1703-1709, XP005298087 ISSN: 0956-5663
- PREININGER C ET AL: "Optical fiber sensor for biological oxygen demand" ANALYTICAL CHEMISTRY, vol. 66, no. 11, 1 June 1994 (1994-06-01), pages 1841-1846, XP000454572 ISSN: 0003-2700 cited in the application

## Description

### FIELD OF THE INVENTION

The present invention relates essentially to a measuring cell, to an analyser comprising said cell, to a process, to a computer program and to a support for said program, to measure, preferably in-situ and in a continuous manner, the Biochemical Oxygen Demand (BOD) of a substance so as to, for instance, estimate the presence of organic matter in said substance, such as for example the water of lakes or rivers, wastewater or products of industrial processes.

### PRIOR STATE OF THE ART

The presence of dissolved oxygen is essential for the life of the aerobic aquatic flora and fauna, as their survival depends on the presence of certain minimum concentrations of this vital substance in the water. However, in lakes, lagoons, rivers, etc., in the proximity of highly urbanised or industrialised areas there is often a decrease in the concentration of dissolved oxygen due to the considerable pollution of their waters. The main cause of the deoxygenation of the water can be attributed to the presence of substances that, as a whole, are denominated "oxygen demanding waste". These are constituted by compounds that are degraded or decompose easily due to the activity of aerobic bacteria. These bacteria use these as food, giving rise to a decrease in the concentration of dissolved oxygen as a consequence of their own respiration.

The test that has been traditionally used to determine the extent in which the organic matter present in water is degraded is that know as five-day Biochemical Oxygen Demand (BOD₅). Said method is slow as it is carried out in the laboratory and requires 5 days before results are obtained.

A recently developed alternative consists in the use of fibre-optic chemical sensors (generally known as FOCS), that allow to perform more rapid measures of the BOD. Known FOCS are generally based upon the measure of luminescence. In general terms, in order to make them work, the radiation from a light source is first sent through a wavelength selector. The radiation is transported via an optical fibre to a reactive layer. The reactive layer is formed by a polymer doped with a suitable chemical indicator, so that an optical property of the indicator experiences a measurable change when a selective interaction with the substance to be detected occurs. The reactive layer modifies the incident light and the modified radiation is sent via an optical fibre to a photodetector. The signal obtained is amplified, analysed and compared with the light emitted initially. The differences between the two permit to deduce the concentration of the analyte under study.

Biosensors, that can be seen as a particular type of FOCS, are another alternative. In the case of a biosensor, the reactive layer is coated with a second layer containing a biomolecule or a biological mass (for example an enzyme, an antibody, bacteria or cells) capable of specifically recognising the substance of interest. As a result of the interaction between the biological layer and the substance being studied, a reaction occurs that is detected by a sensitive element that contains the chemical indicator.

Whether reliable results are obtained or not depends, to a large extent, on the design of the biosensor and of its components, as well as on the method to manufacture it.

In Wolfbeis, O.S. et al., Anal. Chem., 1994, 66, 1841-1846 a biosensor is described for measuring BOD in wastewater which comprises a) a layer sensitive to oxygen that comprises ruthenium (II) tris(4,7-diphenyl-1,10-phenanthroline) perchlorate immobilised on polyester; b) a biological layer that comprises *Trichosporon cutaneum* immobilised upon polyvinyl alcohol; and c) a third layer of polycarbonate to retain the bacterial cells. The response times obtained with said sensor device vary between 5 and 10 minutes with an dynamic BOD range comprised between 0 and 110 mg/L.

In Orellana, G.; Moreno-Bondi, M.C. et al. Anal. Chem., 2001, 73, 5150-5156 a description is made of a photosensitive layer of ruthenium (II) tris(4,7-diphenyl-1,10-phenanthroline) immobilised on silicon and coated with a co-polymer layer based on dodecyl methacrylate and an acrylic monomer containing phosphorylcholine, which reduces the adhesion of marine bacteria and thrombocytes to the surface of said photosensitive layer.

In GB2014979 is described an apparatus for determining biochemical oxygen demand in a substance. In particular, the apparatus comprises, a flow circuit adapted so as to enable the substance to flow through it, said circuit comprising a collection of at least one type of bacteria that interact with the substance decreasing the oxygen concentration in the substance, wherein, the circuit comprises a series of supports colonised by said bacteria, the circuit being adapted for the substance to enter into contact in a necessary and consecutive manner with each support, and, chambers connected to each other by ducts, each support being within a chamber.

In SENSORS AND ACTUATORS B, vol. 110, no. 2, 14 October 2005, pages 289-298, KWOK et al, "An optical biosensor for multi-sample determination of biochemical oxygen demand (BOD)" is described a measuring system of discreet samples showing the use of oxygen sensors of optodes with a particular parallel arrangement.

In BIOSENSORS & BIOELECTRONICS, vol. 21, no. 9, 15 March 2006, pages 1703 - 1709; Lin et al, "Novel BOD optical fibre biosensor based on co immobilized microorganisms in ormosils matrix" is also described a measuring system of discreet samples showing the use of oxygen sensors of optodes.

As can be deduced from the state of the art, several variables have to be taken into account in the development of a biosensor to measure BOD, for instance, the photosensitive species, the matrix upon which immobilisation is going to be performed and the way in which this immobilisation will be done, as well as the nature of the biomass used, being of enormous relevance the design and disposition of the different elements that constitute the measuring device. Variations in any of said variables can lead to an improvement or a loss in the functioning of the FOCS.

Thereby the object of the present invention is to provide an improved analyser and BOD measurement process, allowing its use *in situ* and in a continuous mode, in environmental and industrial applications.

### SUMMARY OF THE INVENTION

A first aspect of the invention refers to a measuring cell to measure the Biochemical Oxygen Demand (BOD) in a substance, which comprises a flow circuit adapted so as to allow the substance to flow through it, said circuit comprising a set of at least one type of bacteria that interact with the substance, decreasing the oxygen concentration of the substance.

According to this first aspect of the invention, this cell is characterised in that the circuit comprises a series of polymeric supports colonised by said bacteria, the circuit being adapted so as to allow the substance to come into contact with each polymeric support in a necessary and consecutive manner. Preferably the substance passes through each colonised polymeric support. In general terms the substance has to be sufficiently liquid so as to be able to flow through the cell, in the knowledge that in the scope of this invention, this substance may be, for example, water loaded with solid debris, a thick sludge or the product of an industrial process such as for instance, the manufacture of beer.

Preferably, the bacteria used to colonise said polymeric support are:
o of the *Pseudomonas putida* type or
o bacteria present in the substance to analyse or
o combinations of bacteria of the *Pseudomonas putida* type and bacteria present in the substance to analyse.

Thereby, advantageously, a cell according to the invention is divided into discrete reaction units (one unit for every polymeric support). Preferably, each discrete reaction unit is the space occupied by each polymeric support colonised with bacteria, to which its immediately surrounding environment could be added, depending on the embodiment.

Excepting for the entry and exit of the substance analysed, that is the beginning and the end of the circuit within it, the cell is an airtight assemblage, without any intermediate entry of oxygen or solutions. A cell according to the present invention offers the following advantages in comparison with a cell according to the prior state of the art (that is one containing a single point-wise biosensor):
- the measurement range for the degradable organic matter is expanded. This is achieved by virtue of the concatenation of successive chambers through which the sample passes (and is degraded) sequentially, making it possible to compartmentalise the reaction into several discrete blocks which can be observed individually,
- it allows the sensitivity of the analyser to be adapted as a whole to the organic load of the substance, by means of a computer programme that takes into consideration the individual results of the measurements performed in each of the chambers being monitored. This makes it possible to guarantee a high precision at low as well as at high concentrations of organic matter and offers additional advantages related to the treatment of the multiple measures.

This system of measures performed by compartments provides the analyser with great flexibility, unavailable in devices based upon a single point-wise biosensor according to the prior state of the art. Thereby this allows analysers comprising a cell according to the present invention to maintain a high level of sensitivity and to guarantee a wide concentration measurement interval.

The reason due to which the compartmentalisation concept implemented by means of this design constitutes the optimal solution is a consequence of the physical nature of the reaction medium itself, that is, of the polymeric support colonised with bacteria: as this colonised polymeric support is solid and static, the set formed by the support together with the substance that traverses it departs completely from the concept of "(quasi) instantaneous mixture" that takes place, for example, in a stirred biological reactor, in which the substrate and the biological catalyst are both dissolved or suspended in a liquid medium.

Considering that the substance travels along a path defined by the circuit of the invention, going across sections of polymeric support (also known as colonised membrane) colonised by immobilised bacteria, for a given sample, and under constant volume flow conditions, the degradation of the sample takes place sequentially and by sections, in a manner related to the location of the solution at any given moment along the circuit, also termed "reaction path". Additionally, by virtue of this, a high total reaction surface area is provided within the circuit.

Thereby, when it becomes necessary to measure a sample with a very low organic load, the high surface area of the polymeric support (the total provided by all of them) enables to make a measurement performed by the whole of the cell, yielding a high sensitivity. Without changing the configuration, when a sample with a high organic load is introduced, the measure of what takes place in the first reaction chamber allows to perform an analytically useful measure within the linear measurement range of dissolved oxygen, before complete deoxygenation of the sample occurs as a consequence of the excess of organic substrate, which would be the unavoidable consequence if an analyser based upon a single, large membrane-bearing sensor placed in the reaction path, were to be used.

As is quite obvious, putting together both characteristics - high sensitivity and the ability to deal with samples with both low and high organic content - is an advantage of a cell according to the present invention. A cell of these characteristics makes it possible to construct an analyser that is of use in the automatic, unattended (for example *in situ*) and continuous measurement of the BOD.

According to an embodiment of the present invention, the circuit comprises chambers, connected to each other by means of ducts, being each polymeric support located in a chamber. The ducts can be preferentially cylindrical and the chambers can be preferentially oval-shaped. The polymeric supports are housed within the chambers.

In an embodiment of the present invention, the circuit comprises at least two layers sensitive to oxygen, being at least the first of these layers superimposed upon the first polymeric support of the series, and being the second of the aforementioned layers superimposed upon the last polymeric support of the series. By the action of these oxygen-sensitive layers, an indication is obtained of the amount of oxygen present in the polymeric supports containing immobilised bacteria, upon which said oxygen-sensitive layers are superimposed.

According to an embodiment, each oxygen-sensitive layer comprises an indicator that has some optical property that varies as a function of the oxygen concentration, preferably the luminescence of the indicator. An example of such an indicator could be a salt of ruthenium (II) tris(4,7-diphenyl-1,10-phenanthroline) or a salt of ruthenium (II) tris [4,7-di(4-byphenylyl)-1,10-phenanthroline] or a combination of both of these salts. In these cases the optical characteristic is that of luminescence.

Preferably, each oxygen-sensitive layer is in turn an assembly of two sub-layers, that comprises another silicone polymeric support and an indicator, where such silicone-based polymeric support comprises (i) a first sub-layer made of silicone that comprises amorphous silica, and (ii) a second sub-layer made of a silicone that covers said first sub-layer.

In an embodiment, the measuring cell has at least two cavities, being the bottom of each cavity sealed by a sealing element, being said cavities adapted so as for each of them to be able to house, at least, one end of an optical fibre thus enabling the collection, via the respective sealing element, of the variations in the optical characteristic of the indicator of the respective oxygen-sensitive layer. Preferably, the sealing element between each discrete reaction unit and the end of the corresponding optical fibre is a layer (for example made of plastic) that is transparent or translucid at least to the wavelengths of the radiations that are going to traverse said layer and that are of interest to measure the BOD, said transparent or translucid layer sealing the cavity.

A second aspect of the present invention refers to an analyser to measure the biochemical oxygen demand in a substance that comprises a flow circuit adapted so as to enable the substance to flow through it, said circuit comprising a set of at least one type of bacteria that interact with the organic matter present in the sample and thereby reducing the dissolved oxygen concentration in the sample.

According to this second aspect of the invention, this analyser is characterised in that the circuit comprises a series of polymeric supports colonised by said bacteria the circuit being adapted so that the substance comes into contact in a necessary and consecutive manner with each polymeric support. Preferably, the substances pass through each colonised polymeric support.

It is possible to obtain, using an analyser according to the present invention, an instrument for the automatic, *in situ* measurement of the biochemical oxygen demand. This measurement can be an estimation to predict what the BOD₅ of the same substance would be. These measurements can be made in a continuous fashion with a greater sensitivity and half-life.

According to an embodiment, the analyser comprises a measuring cell according to one of the embodiments of the first aspect of the invention. In this embodiment, when the measuring cell has oxygen-sensitive layers that comprise an indicator that has some optical characteristic that varies as a function of the oxygen concentration, the analyser can comprise detection means to detect the optical signal coming from each oxygen-sensitive layer when the oxygen-sensitive layers are illuminated by a source of light. The light source can be comprised within the analyser or it can be coupled to a wavelength selector. The light source and/or the detection means can comprise an optical fibre to respectively carry the light to each element and/or collect the optical signal coming from each oxygen-sensitive layer.

According to an embodiment, the analyser comprises means for treating the signal to transform the information obtained by the detection means when the latter detects the optical signal coming from each element, into information that can be treated by processing means.

In an embodiment, the analyser comprises hydraulic means to manage the flow of the substance within the circuit.

According to an embodiment, the hydraulic means are designed to manage the flow of a standard solution within the circuit. Preferably this standard solution is formed by glucose and glutamic acid in water.

In another embodiment, the hydraulic means are designed so as to manage the flow of a pH-regulating solution within the circuit.

A third aspect of the invention concerns a process to continuously measure the biochemical oxygen demand of a substance.

This process of the invention is characterised in that it comprises the following steps:
a) flowing through a circuit of an analyser according to any of the preceding embodiments, at least one regulatory solution to regulate the pH, determining the oxygen concentration, in the proximity of at least the first and the last colonised polymeric supports, referred to as observed polymeric supports, and being said concentrations denominated DORn, being n the position of the polymeric support in the circuit;
b) flowing through the circuit at least a BOD standard solution, determining the oxygen concentration in the proximity of each observed polymeric support, said concentration being referred to as DOSTn;
c) flowing at least the regulatory solution through the circuit until the DORn concentrations are recovered;
d) flowing at least the substance through the circuit, determining the oxygen concentration in the proximity of each observed polymeric support, said concentration being referred to as DOSAn;
e) flowing at least the regulatory solution through the circuit until DORn concentrations are attained again;
f) determining for each polymeric support the oxygen consumption of the substance and of the standard solution, respectively, calculating, on one hand the differences between DOSAn and DORn and those between DOSTn and DORn on the other;
g) Determining the biochemical oxygen demand of the substance calculating the mean of the unit values of biochemical oxygen demand calculated for each of the observed polymeric supports obtained by comparing, for each polymeric support, the oxygen consumption of the substance and of the standard solution obtained in f) with previously obtained calibration values.

The mean of step f) can be weighted. Preferably, the regulatory solution is formed by organic and/or inorganic substances that have the ability of regulating the pH in the range between 5.5 and 9.5, preferably dissolved in a concentration yielding an ionic strength equivalent or below that of 2% sodium chloride.

Preferably, the regulatory solution is found diluted in water in steps a), c) and e), the standard solution is diluted in regulatory solution in step b) and the substance is diluted in regulatory solution in step d).

The regulatory solution can be prepared, for example, by carefully adjusting a 0.01 M solution of sodium phosphate to pH = 6.8, said solution containing an approximate amount of N-allylthiourea of 1 mg L⁻¹, which allows to control the undesired growth of bacteria and avoids interferences in the measure due to the action of nitrifying bacteria. Preferably, the standard solution is formed by one or several substances dissolved in an aqueous medium, that yield a BOD value that is known and stable in time under the diverse working conditions.

By virtue of this aspect of the invention, a process is obtained to measure automatically and *in situ,* the BOD of a substance or of a sample containing, or suspect of containing, organic matter, preferably using an analyser according to any of the embodiments of the first aspect of the invention.

In one of the embodiments of the invention, steps a) to e) are repeated alternating the introduction into the circuit of the substance the BOD of which is to be determined, being it possible to repeat steps c) to e) several times within a same cycle (set of steps
a) to e)). Advantageously, by virtue of this embodiment, a more precise measure of the BOD is obtained.

According to an embodiment of the invention, the mean calculated in step g) is weighted, being the weight: (i) greater for the values calculated in the last observed polymeric supports of the circuit if the substance has a BOD smaller than the BOD of the standard solution, and (ii) smaller for the values calculated in the last observed polymeric supports of the circuit if the substance has a BOD greater than the BOD of the standard solution.

Advantageously, the weighting of the calculated values permits the measuring interval of the system to be enormously extended in addition to providing a far greater reliability to the results obtained.

A fourth aspect of the invention relates to a computer program, characterised in that it comprises computer code means to carry out a process to continuously measure the biochemical oxygen demand of a substance according to an embodiment of the third aspect of the invention, when said program is run on a computer.

In another embodiment, the computer program is copied onto a computer-readable support.

A fifth aspect of the invention concerns a computer-readable support characterised in that in contains a computer program that comprises program code means to carry out a process to continuously measure the biochemical oxygen demand of a substance according to an embodiment of the third aspect of the invention, when said program runs on a computer.

### BRIEF DESCRIPTION OF THE FIGURES

In order to complement the description being made and with the object of providing assistance in obtaining a better understanding of the characteristics of the invention, according to an example of a preferred and practical embodiment of the latter, a set of drawings is provided, as an integral part of said description, in which, with an illustrative and non-limiting purpose, the following have been represented:
Figure 1.- Shows a schematic representation of three views of a measuring cell according to an embodiment of the present invention.
Figure 2a.- Shows a schematic representation of a detail of a measuring cell according to an embodiment of the present invention.
Figure 2b.- Shows a schematic representation of a circuit within a measuring cell according to an embodiment of the present invention.
Figure 3.- Shows a schematic representation of the blocks of an analyser according to an embodiment of the present invention.
Figures 4a and 4b - Show results obtained following a process according to the invention.

### DETAILED DESCRIPTION OF THE INVENTION

In an embodiment, an analyser according to the invention, comprises a measuring cell according to the invention, also referred to as a flow cell, within which a circuit is configured so that the substance the BOD of which is to be measured can flow within it.

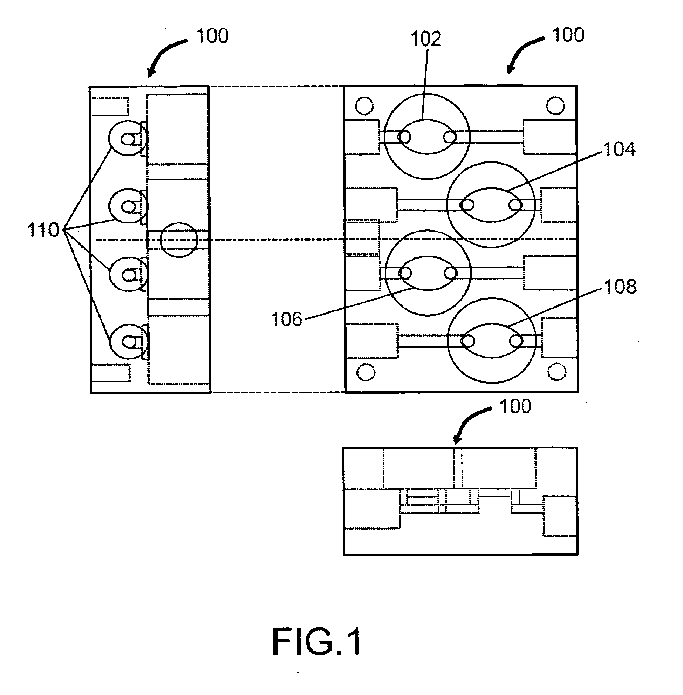

Figure 1 shows schematically three views of a measuring cell according to an embodiment of the present invention.

Measuring cell 100 is substantially, in one embodiment, a rectangular parallelepiped which, as an example, may have the following dimensions: 60 mm of length, 40 mm of width and 22 mm of height.

Preferably, the cell is made in a material having a good thermal conductivity (especially in the event the cell is in an environment in which the temperature is controlled and maintained constant so as to particularly safeguard the biomass present in said cell) in order to favour its thermostatization, that will not rust and that can be mechanised. This material can be duraluminium or stainless steel.

In order to form the circuit through which the different dilutions flow, oval-shaped chambers 102, 104, 106 and 108 can be carved on a wide side (referred to as top) of the rectangular parallelepiped.

To relate the chambers to each other ducts can be drilled from the sides of the rectangular parallelepiped, that span its width, thereby generating 8 threaded perforations 110, that can have a gauge M15 x 1,5 mm ¼" x 28 threads per inch (each chamber having one entry and one exit duct, being the respective entry and exit pair of ducts of each chamber parallel to each other). The oval-shaped chambers are provided to house colonised polymeric supports, connected to each other by cylindrical ducts of a diameter comprised preferentially between 1.0 and 1.5 mm.

Threaded screws of a ¼ of an inch and 28 threads per inch, of the type known as FIA (Flow Injection Analysis) that contain Teflon (polytetrafluoroethylene) tubes can be used to communicate the different ducts.

Some of these polymeric supports are termed "observed" because in these it is possible to observe the oxygen concentration of the material that traverses them. The chambers that contain the observed polymeric supports are also referred to as observed chambers.

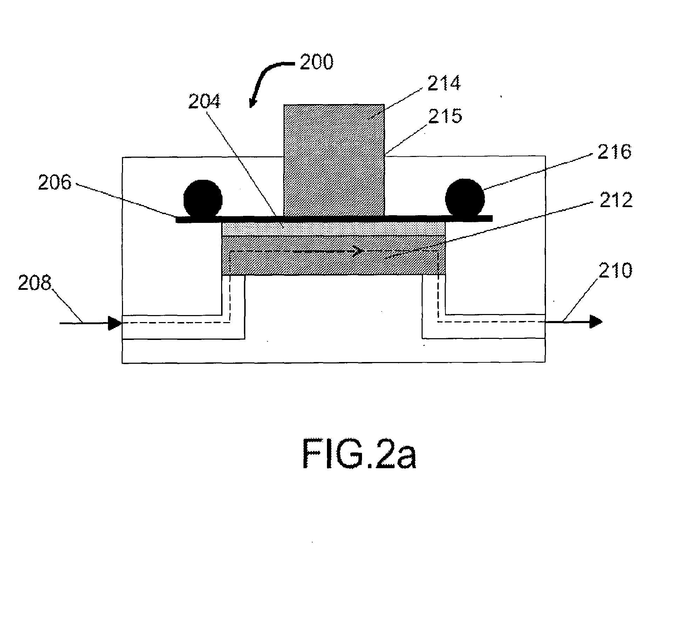

The chambers that are not observed are covered with a stopper, preferably solid, opaque and threaded, made for example of PVC (polyvinyl chloride) or aluminium. The observed chambers are covered with a sealing element preferably at least partially transparent, as can be seen in figure 2a, which shows a schematic representation of an observed chamber 200, that is, an observed reaction unit.

Incoming flows 208 of substances the BOD of which is to be determined, of regulatory solutions or of standard solution can flow along said chamber 200. Those incoming flows pass through the inside of a polymeric support 212 to interact with a biomass, which comprises bacteria, contained in polymeric support 212.

In one embodiment, these bacteria are of the type *Pseudomonas putida.* The culture of *Pseudomonas putida* can be made starting from a lyophilised culture of said microorganism that is cultured under stirring in a liquid medium, in the presence of the support to be colonised. Subsequently, the level of colonisation can be increased by introducing said polymeric support comprising the culture of *Pseudomonas putida* in a flow cell of the measuring device (see below for a description of the measuring device) and re-circulating the nutrient solution through it.

The referred bacteria can be commercially obtained as a lyophilisate of the *Pseudomonas putida* reference strain [code CECT 324 of the Colección Española de Cultivos Tipo, Universidad de Valencia (Spanish Type Culture Collection, University of Valencia)], or from other collections such as ATCC (American Type Culture Collection, ATCC 12633; ATCC 23467, etc....).

Alternatively, it is possible to carry out an uncharacterised mixed culture starting from bacteria that exist in the samples to be measured. For instance, if the BOD of a wastewater sample is to be measured, it is possible to extract from said wastewater, and subsequently obtain a culture, the bacteria that will later be employed as bacterial biomass to colonise the polymeric support.

Said polymeric support containing bacterial biomass must be permeable to the solution that is to be measured. Some useful polymeric supports are, among others: polyvinyl alcohol (PVA), self-curing resins, acetylcellulose, polycarbonate, agarose gel, calcium alginate and Teflon. (K. Riedel, "Application of Biosensors to Environmental Samples" in Commercial Biosensors: Applications to Clinical, Bioprocess and Environmental Samples, G. Ramsay (ed.), Wiley, New York (USA), 1998).

In an embodiment, said first support is comprised by a silicone rubber which is commercially available (for example, ImmobaSil^{®}).

In another embodiment, a polymeric support is obtained starting from an aliquot of a frozen culture, or from a colony isolated on a solid medium, of *Pseudomonas putida:* a culture of this bacterium is prepared, under sterile conditions, in a flask containing 30 mL of Y-1375 liquid broth (SIGMA), and with stirring, in the presence of several units of the polymeric support (for example ImmobaSil^{®} of Ashby Scientific Ltd.) for 24 h at 28 °C. Once the desired level has been achieved, the level of colonisation is further increased by introducing the support in a flow cell and circulating the nutrient solution through it.

An oxygen-sensitive layer 204 lies in intimate contact on top of polymeric support 212.

These oxygen sensitive layers comprise a second polymeric support made of silicone and an indicator, wherein:
a) said silicone-based polymeric support comprises (i) a first silicone sub-layer that comprises amorphous silica, and (ii) a second sub-layer of a silicone that covers said first layer; and
b) said indicator is a Ru(II) complex selected from among the salts of ruthenium (II) tris(4,7-diphenyl-1,10-phenanthroline), of ruthenium(II) tris[4,7-di(4-biphenylyl)-1,10-phenanthroline] and combinations thereof.

The first sub-layer can be made from commercial silicone (for example Dow Corning 3140), modified with amorphous silica of SIGMA. The general composition of these silicones is poly(dimethylsiloxane) and amorphous silica. The second silicone sub-layer can also be accessible commercially (for example Dow Corning Grey 7091) its function being that of protecting the first silicone layer. In order to introduce the indicator, the method described by J. Delgado, PhD Thesis, Universidad Complutense de Madrid, 2000, can be used.

The coordination species of ruthenium (II) and of other transition metals are luminescent dyes that can be used as indicators of the level of oxygen. The spectroscopic properties (absorption and emission energies, emission kinetics) and redox properties of said species can be adjusted to the specific requirements of each case by modifying their ligands. An indicator having the suitable sensitivity to the analyte under study is found for each specific case. The indicators must also exhibit a strong absorption at the desired wavelength, a sufficiently wide Stoke's shift and a half-life of the excited state that will allow to obtain reliable measures.

In order to obtain an oxygen-sensitive layer, it is possible, for example, to prepare the ruthenium (II) tris(4,7-diphenyl-1,10-phenanthroline) following the method described by G. Orellana, C. Álvarez-Ibarra and M. L. Quiroga, Bull. Soc. Chim. Belg. 1988, 97, 731-741. The previously prepared ruthenium (II) tris(4,7-diphenyl-1,10-phenanthroline) is immobilised upon a sheet of commercial silicone (Dow Corning 3140) following the procedure described by J. Delgado, PhD Thesis, Universidad Complutense de Madrid, 2000.

In some embodiments, said indicator comprises a salt of ruthenium(II) tris[4,7-di(4-biphenylyl)-1,10-phenanthroline] synthesised, for example, according to the method described by J. Delgado, PhD Thesis, Universidad Complutense de Madrid, 2000.

In some embodiments it is also possible to use combinations of ruthenium(II) tris(4,7-diphenyl-1,10-phenanthroline) and of ruthenium(II) tris[4,7-di(4-biphenylyl)-1,10-phenanthroline].

The reaction unit or measuring chamber is sealed (ensuring its airtightness) using a sealing element that includes a spacer 206, that is optically transparent (manufactured, for example, in quartz, glass, plastic, poly(methylmethacrylate) or polyester) and an o-ring seal 216. Thereby, the dead volume of the measuring chamber is nil and the solutions of the substance 206, the BOD of which is to be measured, diffuse through the membrane 212 facilitating the interaction between the substances 208 and the biomass of membrane 212.

An end 214 of an optical fibre is mounted passing through a cavity 215 of the upper part of cell 200 so as to reach optical spacer 206 thereby being able to supply the necessary light and, at the same time, observe reaction unit, by collecting the luminescence from the oxygen sensitive layer.

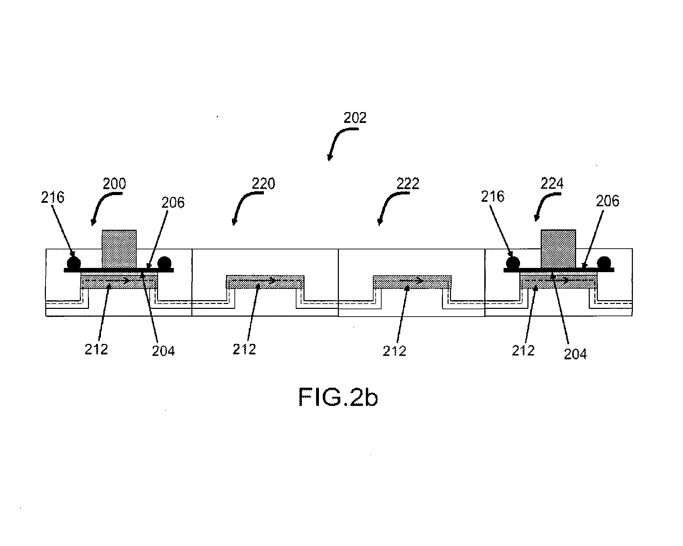

Figure 2b shows an embodiment of a measuring cell 202 to be included in an analyser according to the present invention (which can also be referred to as aerobic bacterial respiration monitoring system). The measuring cell 202 comprises 4 chambers 200, 220, 222 and 224.

Polymeric supports 212 of biocompatible silicone, also referred to as membranes or biomembranes, that contain the immobilised microorganisms, are placed in each of the 4 chambers 200, 220, 222 and 224.

Thereby, this measuring cell comprises in its interior a set of polymeric supports, arranged in line, colonised with a bacterial biomass, as described previously in the description of figure 1.

Chambers 200 and 224 cooperate with the end of an optical fibre, there being oxygen-sensitive layers 204, that have already been previously described, in intimate contact upon their polymeric supports 212.

The design of the flow cell is such that the totality of the volume that flows through it traverses necessarily, and in a consecutive manner, each one of the colonised polymeric supports once these have been incorporated into the oval-shaped chambers of the cell. In this manner, when the solution exits from one chamber it carries a concentration of dissolved oxygen lower or equal to the concentration it contained upon entering it. Each chamber constitutes a reaction unit.

A polymer colonised with a bacterial biomass (of the type already described for figure 1) is housed in each chamber, and an oxygen-sensitive layer is placed in the first and in the last of the chambers of the series (these being the observed chambers).

A device or an analyser to measure the biochemical oxygen demand in a substance or sample comprises, in an embodiment, a multichannel equipment for phase-resolved luminescence measurement.

This multichannel equipment comprises:
(i) a light source coupled to a wavelength selector,
(ii) means of connection, comprising optical fibre between the wavelength selector and the oxygen-sensitive reactive layers,
(iii) a photonic detector, coupled to a wavelength selector, to detect the optical signal coming from the oxygen-sensitive reactive layers and the changes caused therein due to the respiration of the bacterial colonies and
(iv) an electronic equipment to convert the optical signal into an electrical signal and to subsequently process it so as to obtain an analytically useful signal.

The phase-resolved luminescence measuring equipment, to determine the oxygen concentration by interrogating the polymers sensitive to O₂, is preferably composed of three fundamental blocks. Said blocks are the following:
- A. Excitation signal generation system.
- B. Emission signal reception system.
- C. Optical components.

### A. Excitation signal generation system.

The system that permits the generation of the signal that feeds the light source that illuminates the oxygen-sensitive layer is formed by two essential parts:
- Digital part. The main component is a digital oscillator that is in charge of generating the synthetic sinusoidal signal that modulates the light intensity of the excitation source. The frequency of this signal is adjustable between 20 and 140 KHz;
- Analog part. Takes care of adapting the signal levels generated in the digital step, adjusting them to the signal necessary to feed the excitation source. A high intensity blue light emitting diode is used as a light source, which is modulated within the limits of the linear response of the diode.

Both parts are connected by an analog-to-digital converter (A/D) which allows to generate the analogue sinusoid from the synthetic function that is provided by the digital oscillator.

### B. Signal reception system.

As in the case of the generation system, the signal reception block is composed of two parts:
- Analog part. The components included in this part take care of detecting the luminescence coming from the sensitive terminal via the optical fibre, employing for this a Hamamatsu photosensitive module (H6780) that incorporates a miniaturised photomultiplier tube. Likewise this part is in charge of performing a first filtering of the signal to reduce the noise. To do so it has a signal conditioning block and a block with four electronic band-pass filters;
- Digital part. After the analog system follows an analog-to-digital converter that makes it possible to digitise the signal for its subsequent treatment. Subsequently comes a signal integrator that takes care of performing a first processing of the signal, making n averages between the signal received and the signals accumulated before, thereby bringing about an enormous reduction in noise. The samples taken are subsequently processed using an accumulator and decimator resulting in an even greater noise reduction.

The object of this system is, together with that of acquiring the signal emitted by the luminescent indicator, that of obtaining the phase-shift of the signal with respect to the modulated excitation signal. The simplest way of obtaining this phase-shift is carrying out a synchronous demodulation of the signal received (emission) with respect to the original signal. The sinusoidal signal generated (excitation) is converted into a cosine function (excitation + 90°) to obtain the tangent of the phase-shift between the latter and the signal received (emission) by taking the quotient of both functions. As a consequence of the accumulated delays that are caused upon the original signal by the devices contained in the electronic circuit, the signal that strikes the luminescent compound differs, in a certain phase-shift, from the signal at the exit of the digital-to-analogue converter that feeds the emitting diode. Likewise, the sinusoid that reaches the digital part of the signal reception system suffers a certain delay or phase-shift with respect to the luminescence detected by the photomultiplier tube. If the signal synthesised originally is taken as reference, the phase-shift obtained as a result of the demodulation of the emission coming from the oxygen indicator will have a contribution coming from the signal conditioning analog circuits.

To avoid or minimise this contribution, the system incorporates a photodiode situated directly at the exit of the excitation LED, receiving the light emitted by it and the response of which is used as reference signal. This signal will suffer the same accumulative delays originated by the electronic components as the signal that falls on the sensitive terminal. In this manner, all the contributions to the total phase-shift brought about by the electronic components, with the exception of the photomultiplier tube, which is excluded from the path made by the reference or calibration signal, are eliminated from the demodulation process.

### C. Optical components.

At the exit of the excitation LED and immediately prior to the optical fibre, the system has an optical filter, that can be a wide-band filter (typically centred at 400 nm) or an interference filter centred at 400 or 420 nm. Likewise, at the entry of the photomultiplier there is a cut-off filter that, depending on the filter used in the excitation, has a cut-off wavelength of 570. 590 or 630 nm.

The system has a bifurcated-bundle optical fibre that drives the excitatory luminous radiation to the oxygen-sensitive layer and the latter, in turn, back to the detection system.

In an embodiment, an analyser also comprises a device to manage the power of all the instrumentation, the corresponding electrical interfaces and the activation of the electrical devices of the hydraulic block.
In an embodiment, the analyser also comprises a hydraulic block that comprises means for collecting and conditioning a simple, a self-calibration system and a disinfection system.

Additionally, in an embodiment, the analyser comprises processing and memory means to store and run a computer programme according to the invention. This program automatically manages an analyser according to the invention (thereby assuring its autonomy) following a process according to the invention. Preferably, this program can calculate results, store them and represent them in numerical or graphical form.

In general terms, the light source emits luminous radiation that comprises different wavelengths. The wavelength selector to which it is coupled is designed so that a suitable wavelength is selected for the excitation of the oxygen indicator. Said light is transported by the connecting means via the optical fibre to the oxygen-sensitive reactive layer. Within the measuring cell, and as a consequence of the changes in the respiration of the bacterial biomass, the excited states of the oxygen indicator are altered in their light emission kinetics. As a consequence of this, the light returned by the oxygen-sensitive reactive layer becomes modified.

Said modified light is collected in a photonic detector and is transformed into an electrical signal that is processed so as to obtain an analytically useful signal.

In an embodiment, the flow cell is situated within a hydraulic block. The hydraulic block comprises (i) means for the collection and conditioning of a sample, (ii) a self-calibration system and (iii) a disinfection system.

The sample collection and conditioning means comprise a series of pipes that take the sample to a reservoir and stop the latter from emptying once the sample flow has been cut. These also include temperature regulation means that allow to adjust the temperature of the sample to the temperature of the standard solution and to that of the regulatory solution (see below). Additionally, they comprise an aerator that enables the sample to be saturated with air and, finally they comprise a set of pipes, peristaltic pumps and electrovalves that make it possible to modify the sample by dilution, thereby bringing it in optimal condition to be measured into the measuring cell.

The self-calibration system comprises a series of pipes, peristaltic pumps and electrovalves.

The disinfection system comprises a UV emission lamp that illuminates the standard solution and another that illuminates the interior of the instrumentation.

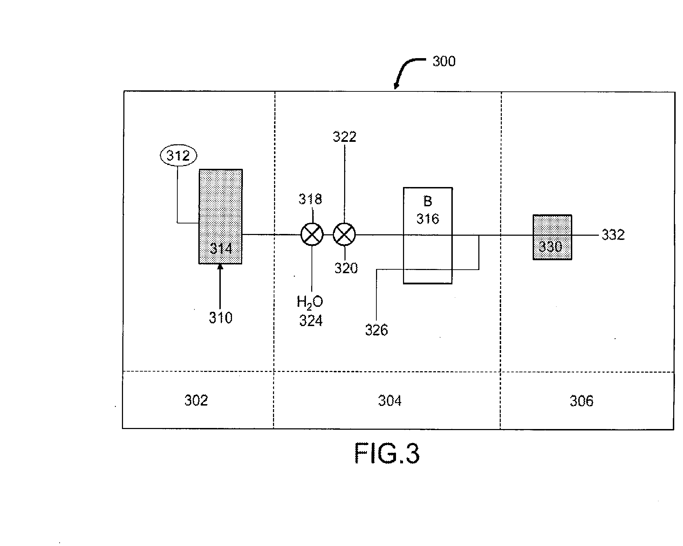

Hydraulic block 300 is represented schematically in figure 3 that shows a functional scheme of an analyser according to an embodiment of the invention. This hydraulic block is divided into three parts: one part 302 for sampling, a part 304 for sample transport and dilution and a part 306 to measure the sample, including measuring cell 330 itself, the temperature of which is controlled by a thermostat, in which the determination of the BOD is performed.

A process, according to an embodiment of the invention, is described hereunder, using for this purpose an embodiment of an analyser according to figures 1, 2 and 3.

The determination of the BOD of substance 310 is carried out following the steps described hereunder.

A flow 310 of the substance the BOD of which is to be determined, referred to as sample, coming from a pumping system, reaches the sampling part 302 of an analyser 300. This pumping system may belong to the measuring device (sump pump or peristaltic pump) as in figure 3 or it can be an independent pumping system that works in cooperation with an electrovalve.

Sample 310 is channelled and stored in a suitable container 314 (such as for example a stainless-steel, PVC or Teflon container) where it is oxygenated by means of an air current coming, for example, from an air compressor 312 with a maximum flow of 2.7 L min⁻¹.

Hydraulic block 300 also comprises, in this embodiment, a part 304 for sample transport and dilution the purpose of which is, by controlling the overall flow so that it remains considerably constant in measuring cell 330, to direct into said measuring cell 330, according to a pre-established sequence, the sample, preferably diluted in regulatory solution, the regulatory solution, that can be diluted with water, or the standard solution, that can be diluted with regulatory solution, as described below.

The different solutions are transported by action of pump 316 (using for example a peristaltic pump as that commercialised by the company Ismatec S.A., Glattbrugg-Zürich, Switzerland) from container 314 to measuring cell 330 in part 306 of sample measurement part 310.

The selection of the standard, regulatory and sample solutions for their pumping into the measuring cell is carried out by switching on / switching off electrovalves 318 and 320 (as for example electrovalves commercialised by the company NResearch Inc., West Caldwell, New Jersey, USA).

With electrovalves 318 and 320 switched off, passage is only permitted to regulatory solution 326 mixed with water 324. By only activating electrovalve 320 the passage of standard 322 is allowed; by adding regulatory solution 326 to measuring cell 330 and only switching on electrovalve 318, passage of the sample to the measuring cell 330 is allowed, adding regulatory solution if necessary. The act of adding regulatory solution to the sample and to the standard solution allows, among other things, to control the volume of flow that reaches measuring cell 330.

In an embodiment, to carry out a routine measurement of the BOD in a sample of surface water (river, lake, etc), regulatory solution 310 is made to flow through the measuring cell for 45 min at a volume of flow of between 0.20 and 0.64 mL min⁻¹ depending on the dilution factor defined by the flows of the pumping system. After said time has elapsed, electrovalve 320 is switched on and standard 322 is circulated through the measuring cell 330, at that same volume of flow, for 20 minutes.

Regulatory solution 310 is again passed through the measuring cell for 45 min and finally, sample 310 is made to pass for 20 minutes through the measuring cell. The calculation of the BOD is performed by means of a computer programme that, additionally, automatically manages all aspects related to the flows that circulate through measuring cell 330.

Waste products 332 can emerge from measuring cell 330.

An example describes the use of an analyser, according to the invention, for the continuous measurement of the BOD. The analyser was installed in the vicinity of the manhole at the exit of the municipal wastewater treatment plant in which sampling was performed, with the aid of a sump pump.
The determination of the BOD of the sample was carried out following a protocol containing the following actions, the set of which is repeated over in time:
- Entry of a regulatory solution (obtained adjusting to pH 6.8 a solution of 0.01 M sodium sulphate that contains an approximate amount of 1 mg L⁻¹ of N-allylthiourea) for a previously determined fixed time t1;
- Entry of a standard solution of glucose and glutamic acid (referred to as GGA and which is described in the standard UNE-EN 1899: Sept. 1998 - Determination of the Biochemical Oxygen Demand after n days (BODn)) of a fixed BOD concentration of 20 mg L⁻¹ diluted in a sodium phosphate solution for a fixed, previously established time t2;
- Repetition for four times of:
   o Entry of the regulatory solution for a time t1:
   o Entry of the sample solution diluted in a solution of sodium phosphate for a time t2.

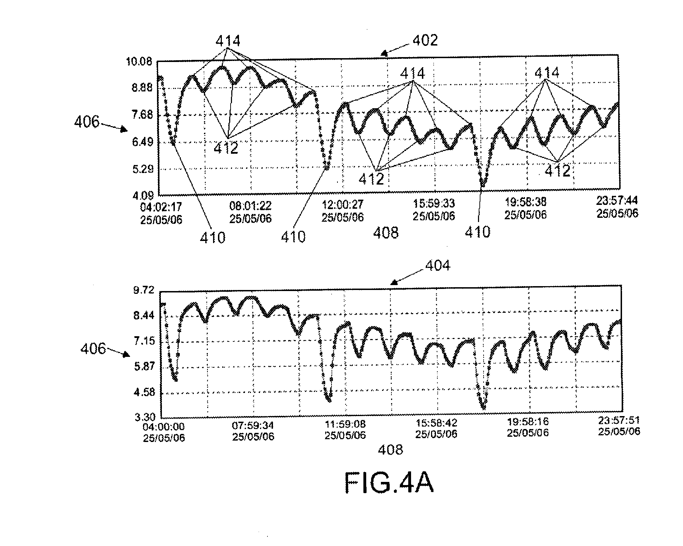

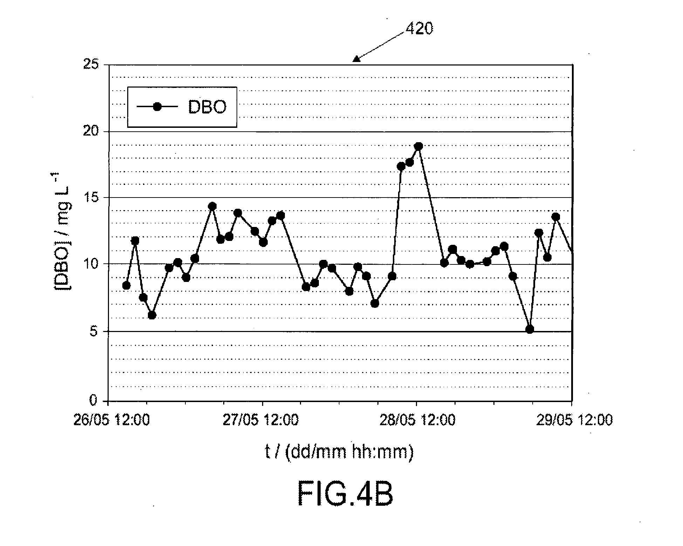

Figures 4a and 4b give a graphical representation of the results obtained. Figure 4a is composed by 2 graphs 402 and 404 which show the evolution of the dissolved oxygen in terms of mg L⁻¹ (ordered as a function of time (hh:mm:ss dd/mm/yy) in the first chamber (graph 402) and in the last chamber (graph 404) of the internal circuit of a measuring cell as that described in figures 1 and 2. The plot represents the measures from consecutive series of 4 samples and a standard.

Figure 4b is a representative plot of the BOD (called DBO in the figure) calculated after the measurement process (calculation corresponding to step f) of a process according to the invention). The plot represents the results from consecutive series of 4 samples and a standard.

The absence of organic matter in the regulatory solution that flows through the cell brings about an increase in the concentration of oxygen in each of the chambers of the measuring cell, that is quantified by oxygen sensors. On the contrary, when organic matter is present in the carrier solution, be it standard GGA solution or sample, a decrease occurs in the concentration of oxygen, proportional to the organic load present in the solution.

For instance, in the first chamber (graph 402), it is possible to see:
- The dissolved oxygen minima 410 corresponding to the passage of the standard solution.
- the dissolved oxygen minima 412, corresponding to the passage of the sample and
- the maxima 414, corresponding to the passage of the regulatory solution.

In the last chamber (graph 404) it is possible to observe a similar graph (minima corresponding to the flow of standard and sample and maxima corresponding to the flow of regulatory solution) but having an overall lower amount of dissolved oxygen, as part of the organic matter traversing the measuring cell has been consumed in the previous chambers. The oxygen concentration read in the last chamber (in this embodiment it is the fourth) is always lower than that read in the first chamber.

This can be extended to any embodiment of the invention because, being there a series of reaction units, the oxygen concentration value in the proximity of each polymeric support (preferably within each polymeric support as solution flow across its interior) is always lower than in the following reaction unit than in the preceding one, being said difference analytically distinguishable. This is the benefit brought about by the series of chambers, making an analyser better than the prior state of the art.

After this data have been obtained one can proceed, according to steps f) and g) of a process according to the invention, to calculating the BOD of the sample so as to obtain, for example, a graph 420 that shows the evolution in time of the BOD in mg L⁻¹.

In an embodiment, this calculation is carried out by a computer program according to the invention, loaded in processing means contained in the analyser.

This calculation comprises the steps of, first, determining for each polymeric support, the oxygen consumption of the substance and of the standard solution, respectively calculating the differences between DOSAn and DORn on one hand and between DOSTn and DORn on the other; and secondly, determining the biochemical oxygen demand in the substance by calculating the mean, that in this embodiment is a weighted mean (see further along), of the unit values of biochemical oxygen demand calculated for each one of the observed polymeric supports, obtained comparing, for each polymeric support, the oxygen consumption of the substance and of the standard solution obtained in f) against previously obtained calibration values.

The oxygen consumption of a sample (or of a standard) is thereby calculated in this embodiment, for each one of the chambers, as the difference between the concentration of dissolved oxygen measured by the optical sensor in the presence of sample DOSAn (or the standard DOSTn) and that in the presence of the regulatory solution DORn.

The resulting BOD value is a weighted mean of the values calculated for each one of the chambers, being the weight of such weighting greater in the last chambers of the flow cell for samples with BOD values below the BOD of the standard, and lower in these last chambers for samples with BOD values greater than the BOD of the standard. This system of employing a series of colonised supports and oxygen sensors, and of weighting the calculated values, allows the measuring range of the system to be enormously extended, in addition to providing greater reliability to the results obtained.

In this case the value of the sample varies between 5 and 19 mgL⁻¹ of BOD. It can be seen how, by virtue of an analyser according to the invention, it is possible to perform the continuous, *in situ* and automatic measure of the BOD.

## Claims

1. Measuring cell (202, 330) to measure the Biochemical Oxygen Demand (BOD) in a substance (310), which comprises
a flow circuit adapted so as to enable the substance (310) to flow through it, said circuit comprising a collection of at least one type of bacteria that interact with the substance (310) decreasing the oxygen concentration of the substance,
the measuring cell comprising
a series of polymeric supports (212) colonised by said bacteria, and being adapted for the substance (310) to enter into contact in a necessary and consecutive manner with each polymeric support (212), and
chambers (102, 200), connected to each other by ducts, each polymeric support (212) being within a chamber (102, 200).
**characterised in that** the measuring cell comprises at least two oxygen-sensitive layers (204), at least the first (204) of these layers being on top of the first polymeric support (212) of the series, and the second (204) of these layers being on top of the last polymeric support (212) of the series.

2. Measuring cell according to claim 1, **characterised in that** each layer (204) comprises an indicator that has an optical characteristic that varies depending on the oxygen concentration.

3. Measuring cell according to the preceding claim, **characterised in that** it has at least two cavities (215), the bottom of each cavity (215) being sealed by a sealing element (206, 216), the cavities (215) being adapted so as for each of them to be able to house at least one end (214) of an optical fibre for collecting through the respective sealing element (206, 216) the respective variations of the optical characteristic of the indicator of the oxygen-sensitive layer (204).

4. Analyser (300) to measure the biochemical oxygen demand in a substance comprising a measuring cell according to any of claims 1 to 3.

5. Analyser according to claim 4 when dependant from claim 2, **characterised in that** it comprises detection means to detect an optical signal coming from each oxygen-sensitive layer (204) when the oxygen-sensitive layers (204) are illuminated by a light source.

6. Analyser according to the previous claim, **characterised in that** it comprises signal treatment means to transform the information obtained by the detection means when the latter detect the optical signal that comes from each oxygen-sensitive layer (204), into information that can be treated by processing means.

7. Analyser according to claims 4, 5 or 6, **characterised in that** it comprises hydraulic means to manage the flow of the substance in the circuit (202).

8. Analyser according to the previous claim, **characterised in that** the hydraulic means are designed to manage the flow of a standard solution within the circuit (202).

9. Analyser according to claims 7 or 8, **characterised in that** the hydraulic means are designed to manage the flow of a pH-regulatory solution within the circuit (202).

10. Process to continuously measure the biochemical oxygen demand in a substance (310), **characterised in that** it comprises the following steps:
a) flowing at least a regulatory solution (326) through a circuit of an analyser (300) according to any of claims 4 to 9, to regulate the pH, determining the oxygen concentration in the proximity of at least the first and the last of the colonised polymeric supports (212), called observed polymeric supports, the concentration called DORn, the position of the polymeric support in the circuit being n;
b) flowing at least a BOD standard solution (322) through the circuit, determining the oxygen concentration in the proximity of each observed colonised polymeric support, the concentration being referred to as DOSTn;
c) flowing through the circuit at least the regulatory solution (326) until the concentrations DORn are recovered;
d) flowing through the circuit at least the substance (310), determining the oxygen concentration in the proximity of each observed polymeric support, the concentration being referred to as DOSAn;
e) flowing through the circuit at least the regulatory solution (326) until the concentrations DORn are recovered;
f) determining for each polymeric support the oxygen consumption of the substance and of the standard solution, respectively, calculating the differences between DOSAn and DORn on one hand, and between DOSTn and DORn, on the other;
g) determining the biochemical oxygen demand of the substance by calculating the mean of the unit values of biochemical oxygen demand calculated for each one of the observed polymeric supports, obtained by comparing, for each polymeric support, the oxygen consumptions of the substance and of the standard solution obtained in f) with previously obtained calibration values.

11. Process according to claim 10, **characterised in that** steps a) to e) are repeated alternating the introduction of the substance the BOD of which is to be measured in the circuit (202) with the introduction of a solution, being it possible to repeat within the same cycle of steps a) to e) the steps c) to e).

12. Process according to claim 10 or 11, **characterised in that** the mean calculated in step g) is weighted, being the weight: (i) greater for the values calculated in the last observed polymeric supports of the circuit if the substance has a BOD below the BOD of the standard solution, and (ii) smaller for the values calculated in the last observed polymeric supports of the circuit if the substance has a BOD greater than the BOD of the standard solution.

13. Computer program, **characterised in that** it comprises program code means to carry out the process to continuously measure the biochemical oxygen demand in a substance according to any of claims 10, 11 or 12, when said program runs on a computer.

14. Computer program according to claim 13, **characterised in that** it is copied on a computer readable means.

15. Computer readable support, **characterised in that** it contains a computer program that comprises program code means to carry out a process to continuously measure the biochemical oxygen demand in a substance according to any of claims 10, 11 or 12, when said program runs on a computer.

## Patentansprüche

1. Messzelle (202, 330) zum Messen des biochemischen Sauerstoffbedarfs (BOD) in einer Substanz (310), welche umfasst:
eine Fluss-Leitung, die dafür ausgelegt ist, der Substanz (310) zu ermöglichen, durch sie zu fließen,
wobei die Leitung eine Ansammlung zumindest einer Art von Bakterien umfasst, die mit der Substanz (310) interagieren, wobei sie die Sauerstoffkonzentration der Substanz senken,
wobei die Messzelle umfasst
eine Reihe von Polymerträgern (212), die von den Bakterien kolonisiert sind und dafür ausgelegt sind, dass die Substanz (310) in einer notwendigen und aufeinander folgenden Weise mit jedem Polymerträger (212) in Kontakt tritt, und
miteinander durch Verbindungsleitungen verbundene Kammern (102, 200), wobei jeder Polymerträger (212) innerhalb einer Kammer (102, 200) ist,
**dadurch gekennzeichnet, dass** die Messzelle zumindest zwei Sauerstoff-sensitive Schichten (204) umfasst,
wobei zumindest die erste (204) dieser Schichten auf dem ersten Polymerträger (212) der Reihe ist und die zweite (204) dieser Schichten auf dem letzten Polymerträger (212) der Reihe ist.

2. Messzelle gemäß Anspruch 1, **dadurch gekennzeichnet, dass** jede Schicht (204) einen Indikator umfasst, der eine optische Eigenschaft hat, die abhängig von der Sauerstoffkonzentration variiert.

3. Messzelle gemäß dem vorherigen Anspruch, **dadurch gekennzeichnet, dass** sie zumindest zwei Höhlungen (215) aufweist, wobei der Boden jeder Höhlung (215) durch ein Dichtelement (206, 216) abgedichtet ist, die Höhlungen (215) dafür ausgelegt sind, dass jede davon in der Lage ist, zumindest ein Ende (214) eines Lichtleiters zum Sammeln, durch das entsprechende Dichtelement (206, 216) der jeweiligen Variationen der optischen Eigenschaft des Indikators der Sauerstoff-sensitiven Schicht (204) aufzunehmen.

4. Analysator (300) zum Messen des biochemischen Sauerstoffbedarfs in einer Substanz, umfassend eine Messzelle gemäß einem der Ansprüche 1 bis 3.

5. Analysator gemäß Anspruch 4 bei Abhängigkeit von Anspruch 2, **dadurch gekennzeichnet, dass** er Detektormittel zum Detektieren eines optischen Signals umfasst, das aus jeder Sauerstoff-sensitiven Schicht (204) kommt, wenn die Sauerstoff-sensitiven Schichten (204) durch eine Lichtquelle beleuchtet werden.

6. Analysator gemäß dem vorherigen Anspruch, **dadurch gekennzeichnet, dass** er Signalbearbeitungsmittel zum Umwandeln der durch das Detektionsmittel erhaltenen Information, wenn das Letztere das optische Signal detektiert, das aus jeder Sauerstoff-sensitiven Schicht (204) kommt, in Informationen, die durch Prozessmittel bearbeitet werden können, umfasst.

7. Analysator gemäß Anspruch 4, 5 oder 6, **dadurch gekennzeichnet, dass** er ein hydraulisches Mittel zur Verwaltung des Flusses der Substanz in der Leitung (202) umfasst.

8. Analysator gemäß dem vorherigen Anspruch, **dadurch gekennzeichnet, dass** das hydraulische Mittel dafür entworfen ist, den Fluss einer Standardlösung innerhalb der Leitung (202) zu verwalten.

9. Analysator gemäß Anspruch 7 oder 8, **dadurch gekennzeichnet, dass** das hydraulische Mittel dafür entworfen ist, den Fluss einer pH-regulatorischen Lösung innerhalb der Leitung (202) zu verwalten.

10. Prozess zum kontinuierlichen Messen des biochemischen Sauerstoffbedarfs in einer Substanz (310), **dadurch gekennzeichnet, dass** er die folgenden Schritte umfasst:
a) Fließenlassen zumindest einer regulatorischen Lösung (326) durch eine Leitung eines Analysators (300) gemäß einem der Ansprüche 4 bis 9, um den pH zu regulieren, wobei die Sauerstoffkonzentration in der Nähe von zumindest dem ersten und letzten der kolonisierten Polymerträger (212), die observierte Polymerträger genannt werden, bestimmt wird, wobei die Konzentration DORn genannt wird, wobei die Position des Polymerträgers in der Leitung n ist;
b) Fließenlassen zumindest einer BOD-Standardlösung (322) durch die Leitung, wobei die
Sauerstoffkonzentration in der Nähe jedes observierten kolonisierten Polymerträgers bestimmt wird, wobei die Konzentration als DOSTn bezeichnet wird;
c) Fließenlassen, durch die Leitung zumindest der regulatorischen Lösung (326), bis die Konzentrationen DORn wieder hergestellt sind;
d) Fließenlassen, durch die Leitung, zumindest der Substanz (310), wobei die Sauerstoffkonzentration in der Nähe jedes observierten Polymerträgers bestimmt wird, wobei die Konzentration als DOSAn bezeichnet wird;
e) Fließenlassen, durch die Leitung, zumindest der regulatorischen Lösung (326), bis die Konzentrationen DORn wieder hergestellt sind;
f) Bestimmen, für jeden Polymerträger, des Sauerstoffverbrauchs der Substanz bzw. der Standardlösung, wobei die Differenzen zwischen DOSAn und DORn einerseits, und zwischen DOSTn und DORn andererseits berechnet werden;
g) Bestimmen des biochemischen Sauerstoffbedarfs der Substanz durch Berechnung des Durchschnitts der Einheitswerte an biochemischem Sauerstoffbedarf, die für jeden der observierten Polymerträger berechnet sind, erhalten durch Vergleichen, für jeden Polymerträger, der Sauerstoffverbräuche der Substanz und der Standardlösung, die in f) erhalten wurden, mit zuvor erhaltenen Kalibrierungswerten.

11. Prozess gemäß Anspruch 10, **dadurch gekennzeichnet, dass** die Schritte a) bis e) wiederholt werden, wobei die Einführung der Substanz, deren BOD in der Leitung (202) zu messen ist, mit der Einführung einer Lösung alterniert, wobei es möglich ist, innerhalb desselben Zyklus von Schritten a) bis e) die Schritte c) bis e) zu wiederholen.

12. Prozess gemäß Anspruch 10 oder 11, **dadurch gekennzeichnet, dass** der in Schritt g) berechnete Durchschnitt gewichtet wird, wobei die Gewichtung: (i) größer für die in den letzten observierten Polymerträgern der Leitung berechneten Werte ist, falls die Substanz einen BOD unter dem BOD der Standardlösung aufweist, und (ii) kleiner für die in dem letzten observierten Polymerträgern der Leitung berechneten Werte ist, falls die Substanz einen BOD größer als den BOD der Standardlösung aufweist.

13. Computerprogramm, **dadurch gekennzeichnet, dass** es Programmcodemittel zum Ausführen des Prozesses zu einer kontinuierlichen Messung des biochemischen Sauerstoffbedarfs in einer Substanz gemäß einem der Ansprüche 10, 11 oder 12 umfasst, wobei das Programm auf einem Computer abläuft.

14. Computerprogramm gemäß Anspruch 13, **dadurch gekennzeichnet, dass** es auf ein computerlesbares Medium kopiert ist.

15. Computerlesbarer Träger, **dadurch gekennzeichnet, dass** er ein Computerprogramm enthält, das Programmcodemittel zum Ausführen eines Prozesses zur kontinuierlichen Messung des biochemischen Sauerstoffbedarfs in einer Substanz gemäß einem der Ansprüche 10, 11 oder 12 umfasst, wenn das Programm auf einem Computer abläuft.

## Revendications

1. Cellule de mesure (202, 330) pour mesurer la Demande Biochimique en Oxygène (BOD) dans une substance, qui comprend :
un circuit de circulation adapté à permettre à la substance (310) de circuler à travers lui, le dit circuit comprenant une collection d'au moins un type de bactéries qui interagit avec la substance (310) en diminuant la concentration en oxygène de la substance,
la cellule de mesure comprenant
une série de supports polymériques (212) colonisés par les dites bactéries, et adaptés à ce que la substance (310) entre en contact avec chaque support polymérique (212) d'une façon nécessaire et consécutive, et
des chambres (102, 200), connectées les unes aux autres par des conduits, chaque support polymérique (212) étant à l'intérieur d'une chambre (102, 200),
**caractérisée en ce que** la cellule de mesure comprend au moins deux couches sensibles à l'oxygène (204), au moins la première de ces couches (204) étant sur le premier support polymérique (212) de la série, et la deuxième (204) de ces couches étant sur le dernier support polymérique (212) de la série.

2. Cellule de mesure selon la revendication 1, **caractérisée en ce que** chaque couche (204) comprend un indicateur qui a une caractéristique optique qui varie en fonction de la concentration en oxygène.

3. Cellule de mesure selon la revendication précédente, **caractérisée en ce qu'**elle a au moins deux cavités (215), le fond de chaque cavité (215) étant scellé par un élément de scellement (206, 216), les cavités (215) étant adaptées à ce que chacune d'elle soit capable d'accueillir au moins une extrémité (214) d'une fibre optique pour recueillir à travers l'élément de scellement respectif (206, 216) les variations respectives de la caractéristique optique de l'indicateur de la couche sensible à l'oxygène (204).

4. Analyseur (300) pour mesurer la demande biochimique en oxygène dans une substance comprenant une cellule de mesure selon l'une quelconque des revendications 1 à 3.

5. Analyseur selon la revendication 4 quand elle se rattache à la revendication 2, **caractérisé en ce qu'**il comprend un moyen de détection pour détecter un signal optique provenant de chaque couche sensible à l'oxygène (204) quand les couches sensibles à l'oxygène (204) sont éclairées par une source lumineuse.

6. Analyseur selon la revendication précédente, **caractérisé en ce qu'**il comprend un moyen de traitement du signal pour transformer l'information obtenue par le moyen de détection quand ce dernier détecte le signal optique qui vient de chaque couche sensible à l'oxygène (204), en information qui peut être traitée par un moyen de traitement.

7. Analyseur selon les revendications 4, 5 ou 6, **caractérisé en ce qu'**il comprend un moyen hydraulique pour gérer la circulation de la substance dans le circuit (202).

8. Analyseur selon la revendication précédente, **caractérisé en ce que** le moyen hydraulique est conçu pour gérer la circulation d'une solution standard à l'intérieur du circuit (202).

9. Analyseur selon les revendications 7 ou 8, **caractérisé en ce que** le moyen hydraulique est conçu pour gérer la circulation d'une solution de régulation du pH à l'intérieur du circuit (202).

10. Procédé pour mesurer en continu la demande biochimique en oxygène dans une substance (310), **caractérisé en ce qu'**il comprend les étapes suivantes :
a) faire circuler au moins une solution de régulation (326) dans le circuit d'un analyseur (300) selon l'une quelconque des revendications 4 à 9, pour réguler le pH, en déterminant la concentration en oxygène à proximité d'au moins le premier et le dernier des supports polymériques colonisés (212), appelés des supports polymériques observés, la concentration étant appelée DORn, la position du support polymérique dans le circuit étant n,
b) faire circuler au moins une solution BOD standard (322) dans le circuit, en déterminant la concentration en oxygène à proximité de chaque support polymérique colonisé observé, la concentration étant appelée DOSTn,
c) faire circuler dans le circuit au moins la solution de régulation (326) jusqu'à ce que les concentrations DORs soient récupérées,
d) faire circuler dans le circuit au moins la substance (310), en déterminant la concentration en oxygène à proximité de chaque support polymérique observé, la concentration étant appelée DOSAn,
e) faire circuler dans le circuit au moins la solution de régulation (326) jusqu'à ce que les concentrations DORn soient récupérées,
f) déterminer pour chaque support polymérique la consommation en oxygène de la substance et de la solution standard, respectivement, en calculant les différences entre DOSAn et DORn d'une part, et entre DOSTn et DORn, d'autre part,
g) déterminer la demande biochimique en oxygène de la substance en calculant la moyenne des valeurs unitaires de demande biochimique en oxygène calculées pour chacun des supports polymériques observés, obtenues en comparant, pour chaque support polymérique, les consommations en oxygène de la substance et de la solution standard obtenues en f) avec des valeurs de calibrage obtenues.

11. Procédé selon la revendication 10, **caractérisé en ce que** les étapes a) à e) sont répétées en alternant l'introduction de la substance dont la BOD doit être mesurée dans le circuit (202) avec l'introduction d'une solution, étant possible de répéter dans le même cycle d'étapes a) à e) les étapes c) à e).

12. Procédé selon la revendication 10 ou 11, **caractérisé en ce que** la moyenne calculée à l'étape g) est pondérée, la pondération étant : (i) plus grande pour les valeurs calculées dans les derniers supports polymériques observés du circuit si la substance a une BOD inférieure à la BOD de la solution standard, et (ii) plus petite pour les valeurs calculées dans les derniers supports polymériques observés du circuit si la substance a une BOD plus grande que la BOD de la solution standard.

13. Programme informatique, **caractérisé en ce qu'**il comprend un moyen de codage de programme pour mettre en oeuvre le procédé pour mesurer en continu la demande biochimique en oxygène dans une substance selon l'une quelconque des revendications 10, 11 ou 12, quand le dit programme tourne sur un ordinateur.

14. Programme informatique selon la revendication 13, **caractérisé en ce qu'**il est copié sur un moyen lisible par un ordinateur.

15. Support lisible par un ordinateur, **caractérisé en ce qu'**il contient un programme informatique qui comprend un moyen de codage de programme pour mettre en oeuvre un procédé pour mesurer en continu la demande biochimique en oxygène dans une substance selon l'une quelconque des revendications 10, 11 ou 12 quand le dit programme tourne sur un ordinateur.
